# EUROPEAN PATENT APPLICATION

(11) **EP 4 778 487 A1**
(43) Date of publication of application: **22.07.2026**
(21) Application number: 24864657.2
(22) Date of filing: 11.09.2024
(51) Int. Cl.: A61B 34/20, A61B 34/30, A61B 34/00, A61F 2/44, A61F 2/46

(54) **REFERENCE FRAME FOR ELECTROMAGNETIC POSITIONING NAVIGATION OF ORTHOPEDIC SURGERY ROBOT AND CALIBRATION METHOD THEREFOR**

(30) Priority: 14.09.2023 CN 202311184296
(71) Applicant: CHUNFENGHUAYU (SUZHOU) INTELLIGENT MEDICAL TECHNOLOGY CO., LTD., Taicang Suzhou, Jiangsu 215413 (CN)
(72) Inventor: YOUNG, Victor, Suzhou, Jiangsu 215413 (CN); GUPTA, Shaurya, Suzhou, Jiangsu 215413 (CN); SHI, Weisong, Suzhou, Jiangsu 215413 (CN); CHEN, Chaoliang, Suzhou, Jiangsu 215413 (CN)
(74) Representative: Maiwald GmbH
(86) International application number: PCT/CN2024/118269
(87) International publication number: WO 2025/055941

(57) **Abstract**

A reference frame for electromagnetic positioning navigation of an orthopedic surgery robot, and a method for calibrating the reference frame. The reference frame is designed as an interbody fusion cage device (100) and comprises a fusion cage body (110), which is used for being implanted between two vertebral bodies related to a focus, and is provided with a top surface (122), a bottom surface (123) and side surfaces (124, 125), an opening (111) for filling a bone graft material being formed in the middle areas of the top surface (122) and the bottom surface (123), a positioning sensor device (200), used for electromagnetic positioning navigation of an orthopedic surgery robot, and detachably connected to the interbody fusion cage device (100); and a developing device (121), used for displaying coordinate positions in an image acquired by a medical imaging device and fixedly arranged on the interbody fusion cage body (110).

## Description

For all purposes, the present application claims priority to Chinese Patent Application No. 202311184296.2 filed on September 14, 2023, the entire content of which is incorporated herein by reference as part of the present application.

### TECHNICAL FIELD

The present disclosure relates to a reference frame for electromagnetic positioning and navigation of an orthopedic surgical robot. Furthermore, the present disclosure further relates to a method for calibrating the reference frame.

### BACKGROUND

Robot-assisted orthopedic surgery is an important application of orthopedic surgical robots. In the past, surgeons placed screws in the spinal bone to perform complex spinal surgeries manually or with the assistance of numerous intraoperative X-ray images, resulting in a risk of radiation exposure for both patients and surgeons. Furthermore, spinal surgery requires high precision in screw placement, and the consequences of placing screws in incorrect or suboptimal positions are severe.

Orthopedic surgical robots can provide a guidance system based on computerized preoperative planning, which significantly improves accuracy and reduces the risk of screw misplacement. In conventional orthopedic spinal pedicle screw placement surgery using a robot, a guide wire is inserted according to intraoperative planning, followed by the placement of a hollow pedicle screw through the guide wire; ultimately, the procedure still relies on manual drilling and screw placement by the surgeon, resulting in low screw placement efficiency. Existing orthopedic surgical robots are generally equipped with an infrared navigation system, which is affected by obstruction by intraoperative items and movement of the patient or patient support device, leading to navigation deviations, surgical interruptions and other issues.

The present inventors have developed a reference frame for an electromagnetic navigation device of a surgical robot and an electromagnetic navigation system with the reference frame, which can overcome the disadvantage that the optical navigation of the surgical robot in the prior art is easily blocked by objects and achieve high-precision navigation.

In the prior art, it is known that an infrared navigation system for a surgical robot usually arranges reflective balls for tracking on a separate reference frame, and this reference frame is far from the surgical site, which also places the reflective balls for tracking far away. This arrangement in the infrared navigation system will lead to significant errors in positioning and navigation. Therefore, there is currently a need to find a solution that is suitable for electromagnetic positioning of orthopedic surgical robots and can improve the positioning and navigation accuracy of orthopedic surgical robots.

### SUMMARY

The technical problem to be solved by the present disclosure is to provide a reference frame for electromagnetic positioning and navigation of an orthopedic surgical robot, which can cooperate with an electromagnetic positioning and navigation system to provide real-time and accurate navigation for the operation of the surgical robot.

In one aspect, the technical problem is solved by a reference frame for electromagnetic positioning and navigation of an orthopedic surgical robot, where the reference frame is configured as an interbody fusion device, and the reference frame includes:
a fusion cage body, configured to be implanted between two vertebral bodies associated with a lesion, where the fusion cage body has a top surface, a bottom surface and a side surface, and an opening for filling bone graft material is provided in a middle area between the top surface and the bottom surface;
a positioning sensor device for the electromagnetic positioning and navigation of the orthopedic surgical robot, where the positioning sensor device is detachably connected to the interbody fusion device; and
a marker, configured to display a coordinate position in an image acquired by a medical imaging device, wherein the marker is fixedly arranged on the fusion cage body.

By designing the reference frame as an interbody fusion device, there is no need to additionally install an external reference frame in the orthopedic surgical robot system before, for example, spinal fusion surgery, which can simplify the operation procedure, eliminate the need for equipment designed as a separate reference frame and thus save equipment costs.

In addition, because the reference frame is designed as an interbody fusion device, the positioning sensor device detachably mounted thereon is sufficiently close to the surgical site to be operated on, and the interbody fusion cage is positioned in real time by the electromagnetic navigation and positioning system, avoiding errors caused by the movement of the surgical object and the like, and greatly improving the positioning precision and accuracy.

To obtain accurate registration data, the marker is designed as at least three tantalum markers, which are dispersedly arranged on the fusion cage body as a marking portion, thereby enabling the calculation of a registration matrix from the coordinates of the medical imaging device to the electromagnetic space coordinates. Further preferably, the marker is designed as four tantalum markers, thereby achieving a relatively accurate registration effect. This registration effect also depends on the dispersion degree of the tantalum markers on the fusion cage body; the higher the dispersion degree of the tantalum markers on the fusion cage body, the more accurate the registration effect that can be obtained.

In an extended embodiment, it is provided that the top surface and the bottom surface of the fusion cage body are each at least partially provided with an anti-slip portion for increasing the surface frictional resistance of the contact between the two vertebral bodies and the fusion cage body. Preferably, the anti-slip portion is designed as inverted teeth, which are particularly used to prevent the fusion cage body from slipping out from a position between the two vertebral bodies and are uniformly distributed on the entire top surface and the entire bottom surface of the fusion cage body.

To stably mount the positioning sensor device on the reference frame, especially on the fusion cage body of the interbody fusion device, and in consideration of the convenience of inserting or removing the interbody fusion device between two relevant vertebral bodies, a hole for detachably connecting the positioning sensor device is provided on a side surface of the fusion cage body, where the connection is achieved in a friction fit manner.

In another extended embodiment, it is provided that at least one opening for positioning on a calibration device for calibrating the reference frame is provided on the side surface of the fusion cage body, and the at least one opening is connected with a protrusion on the calibration device in a form-fitting manner, a plug-in manner or a clamping manner.

If only one opening for positioning on the calibration device for the reference frame is provided on the side surface of the fusion cage body, and the cross section of the opening is non-circular, preferably polygonal or elliptical, then the positioning of the reference frame, especially the interbody fusion cage of the present disclosure, by the calibration device for calibration position can be ensured with a minimum number of openings, i.e., one opening, on the side surface of the fusion cage body. This is because the non-circular, preferably polygonal or elliptical cross section of the opening can restrict the rotation of the interbody fusion device mounted thereon around the protrusion through form-fitting connection with the corresponding protrusion on the fusion cage body. The design of only one such shaped opening is particularly advantageous when the fusion cage body is small and the space or position for providing the opening is very limited.

Certainly, two or more openings for positioning on the calibration device for the reference frame may also be provided on the side surface of the fusion cage body, and the cross section of the openings is circular. Openings with such a cross-sectional shape are easier to machine, thus the manufacture of the fusion cage body is correspondingly easier.

In another aspect, the technical problem of the present disclosure is solved by a method for calibrating the above-mentioned reference frame, and the method includes the steps:
S1: providing a calibration device for the reference frame, the calibration device being provided with a positioning device for positioning the reference frame, especially the interbody fusion device, and a calibration sensor device being fixedly mounted on the calibration device, where a position relationship matrix between the positioning sensor device and the marker can be determined by the calibration device to obtain the coordinates of the marker in the electromagnetic space.
S2: positioning the reference frame, especially the interbody fusion device, on the calibration device by the positioning device.
S3: acquiring the coordinates of the positioning sensor device and the calibration sensor device in the electromagnetic space respectively under an electromagnetic field for electromagnetic positioning and navigation.
S4: calculating a transformation matrix from the calibration sensor device to the positioning sensor device based on the acquired coordinates.
S5: determining a position relationship between the calibration sensor device and the marker based on a preset arrangement of the marker and the calibration sensor device in the calibration device.
S6: calculating a position relationship matrix between the positioning sensor device and the marker based on the calculated transformation matrix and the determined position relationship.

As a supplement, to reduce the error of the calculated position relationship matrix and thus further improve the electromagnetic positioning precision of the marker, after step S6, step S7 may be additionally performed: moving the calibration device in the electromagnetic field for electromagnetic positioning and navigation, where the movement may be translation or rotation, but rotation is preferred to obtain a plurality of different poses of the calibration device, where steps S3-S6 are repeated in each pose to acquire a plurality of position relationship matrices between the positioning sensor device and the marker, and an average position relationship matrix is calculated from the plurality of position relationship matrices.

In a further extended embodiment, it is provided that the calibration device has a top side and a bottom side, at least one recess for accommodating at least one reference frame is provided on the top side, a depth of the recess is designed to correspond to a height of the reference frame, and the positioning device is provided in the at least one recess.

In another extended embodiment, it is provided that after step S2 and before step S3, step S2' is additionally performed: pressing the reference frame downward to abut against the calibration device by an additional pressing device and keeping the reference frame fixed to prevent the reference frame, especially the interbody fusion device, from moving along a height direction of the positioning device when the calibration device is moved.

In a further extended embodiment, it is provided that the positioning device is designed as at least one protrusion, a shape of the at least one protrusion and a size of the at least one protrusion are designed to be connected to at least one opening provided on the side surface of the fusion cage body in a form-fitting manner, a plug-in manner or a clamping manner.

If the positioning device is designed as only one protrusion, and the cross section of the protrusion is non-circular, preferably polygonal or elliptical, the positioning of the reference frame, especially the interbody fusion cage of the present disclosure, by the calibration device can be ensured with a minimum number of protrusions, i.e., one protrusion. This is because the non-circular, preferably polygonal or elliptical cross section of the protrusion can restrict the rotation of the interbody fusion device mounted thereon around the protrusion through form-fitting connection with the opening provided on the fusion cage body. The design of only one such shaped protrusion can make the provision of the calibration device easier and thus the calibration process simpler.

If the positioning device is designed as two or more protrusions, the cross section of the protrusions may be circular or annular, and in particular, the protrusions may be designed in the form of positioning pins, such that the protrusions can be machined more easily.

Further details and advantages of the examples described in detail herein will become apparent from the following detailed description of the examples in conjunction with the accompanying drawings.

### BRIEF DESCRIPTION OF DRAWINGS

Fig. 1 is a perspective view of an interbody fusion device according to a non-limiting embodiment of the present disclosure;
Fig. 2 is a perspective view of a fusion cage body of an interbody fusion device according to a non-limiting embodiment of the present disclosure;
Fig. 3 is a perspective view of a calibration device in a calibration method according to a non-limiting embodiment of the present disclosure;
Fig. 4 is a flow chart of the steps of a method for calibrating the reference frame according to the present disclosure.

Referring to the accompanying drawings, where same reference numerals refer to same components throughout the several views, and the present disclosure relates to a surgical robot for orthopedic surgery.

### DETAILED DESCRIPTION

Referring to the accompanying drawings, where same reference numerals refer to same components throughout the several views, and the present disclosure relates to a surgical robot for orthopedic surgery.

The terms "interbody fusion device" and "interbody fusion cage" used herein may be used interchangeably as they basically describe the same instrument. Described herein is an interbody fusion device for electromagnetic navigation of a surgical robot. By implanting the interbody fusion device of the present disclosure into the space between two vertebral bodies of a patient with diseased or damaged intervertebral discs or spines, bone growth between the two vertebral bodies can be achieved to fill the space left after discectomy.

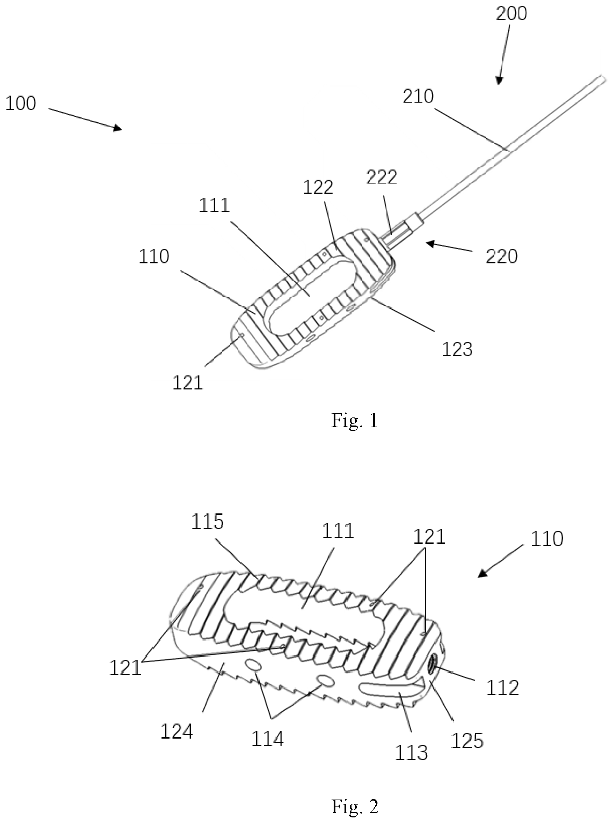

Fig. 1 is a perspective view of an interbody fusion device 100 as a reference frame for electromagnetic positioning and navigation of an orthopedic surgical robot, viewed obliquely from above the interbody fusion device 100. The interbody fusion device 100 according to the present disclosure is designed for registration of data acquired by an electromagnetic navigation system and a medical imaging device, and for intraoperative navigation of the orthopedic surgical robot, while restoring intervertebral space height and physiological curvature, achieving immediate postoperative stability, reducing the occurrence of pedicle screw fracture, promoting interbody bone fusion, and to some extent reducing the amount of autologous bone used, thereby reducing a series of complications arising therefrom. The interbody fusion device 100 includes a fusion cage body 110, a positioning sensor device 200 and a marker 121. The medical imaging device may preferably be designed as a three-dimensional CT scanner, or may be designed as other three-dimensional imaging devices.

The fusion cage body 110 is configured to be implanted between two vertebral bodies associated with a lesion and has a top surface 122, a bottom surface 123 and a plurality of, for example four, side surfaces, namely, two short side surfaces 125 and two long side surfaces 124. An opening 111 for filling bone graft material is provided in a middle area between the top surface 122 and the bottom surface 123. The positioning sensor device 200 is designed for electromagnetic positioning of the interbody fusion cage and may be detachably connected to the fusion cage body 110, so as to stably mount the positioning sensor device 200 on the reference frame, especially on the fusion cage body 110 of the interbody fusion device, and in consideration of the convenience of inserting or removing the interbody fusion device 100 between two relevant vertebral bodies. The detachable connection may preferably be achieved in a friction fit manner, in particular a threaded connection, or a form-fitting manner, in particular a quick-connect manner, and other similar easily detachable connection manners. In a non-limiting embodiment of the present disclosure, the positioning sensor device 200 may include a sensor cable 210, a sensor connector 220, an external thread and an external hexagon 222. The external thread is frictionally engaged with an internal thread of a hole 112 (as shown in Fig. 2) in the fusion cage body 110 for mutual tightening, and the external hexagon 222 is used for cooperation with a removal tool, such as an internal hexagon sleeve, when removing the positioning sensor device 200. The marker 121 is designed for displaying a coordinate position in an image acquired by a medical imaging device and is fixedly arranged on the fusion cage body 110. The fusion cage body 110 will be further described below.

Fig. 2 is a perspective view of the fusion cage body 110 of the present disclosure. The contour of the fusion cage body 110 may be designed to be substantially polygonal, in particular rectangular as shown in Fig. 2, to facilitate the manufacture and processing of the fusion cage body 110. Anti-slip portions 115 are respectively provided on the top surface 122 and the bottom surface 123 of the fusion cage body 110 for increasing surface frictional resistance of the contact between the two vertebral bodies and the fusion cage body. The anti-slip portions 115 may be distributed over the entire surface, particularly uniformly distributed on the entire top surface 122 and the entire bottom surface 123 of the fusion cage body 110, or may be distributed partially, particularly uniformly distributed on a part of the top surface 122 and a part of the bottom surface 123 of the fusion cage body 110. In the present non-limiting embodiment, the anti-slip portions 115 may be designed as inverted teeth, which are particularly used to prevent the fusion cage body 110 from slipping out from a position between the two vertebral bodies and are uniformly distributed on the entire top surface 122 and the entire bottom surface 123 of the fusion cage body 110. Alternatively, the anti-slip portions 115 may also be designed as a convex dot shape or a convex pin shape.

The marker 121 is detectable on the medical imaging device and is designed as four tantalum markers in Fig. 2 for displaying in images acquired by the medical imaging device and providing the position relationship between the tantalum markers and the positioning sensor device 200. The four tantalum markers are dispersedly arranged on the fusion cage body 110, thereby achieving a relatively accurate registration effect. The registration effect also depends on the dispersion degree of the tantalum markers on the fusion cage body 110; the higher the dispersion degree of the tantalum markers on the fusion cage body 110, the more accurate the registration effect that can be obtained. Certainly, the number of tantalum markers may also be three or more than four, and the greater the number of tantalum markers, the better the registration effect achieved by the reference frame of the present disclosure, especially the interbody fusion device 100. The tantalum markers may be implemented in the form of tantalum wires, tantalum rods, tantalum balls or the like.

In the present disclosure, the marker 121, especially the tantalum markers, is preconfigured with a unique identifier according to a pattern of their positions, number and composition, etc., formed on the fusion cage body 110, thereby enabling encoding of information such as the identifier of a unique surgical object, through which the surgical object and its condition can be identified, and also providing information about the tantalum markers and the surgical object, information about the implantation situation and date, the surgeon, the surgical location and the like. Medical information itself may also be directly encoded into the marker 121, or the code on the marker 121 may be manually or automatically input into a corresponding record in a computerized database containing medical information, where the medical information may include data such as pathological reports collected from the marked site, and this information may be input into the computer record before or after implantation of a marking portion.

As can be seen from Fig. 2, the hole 112 for detachably connecting the positioning sensor device 200 is provided on the short side surface 125 of the rectangular parallelepiped fusion cage body 110, which is beneficial for the patient to obtain a smaller surgical incision for implanting the interbody fusion device 100. Certainly, the hole 112 for detachably connecting the positioning sensor device 200 may also be provided on the long side surface 124 of the fusion cage body 110. As shown in the figure, an internal thread is further provided in the hole 112, and through the internal thread, a detachable connection with the external thread of the positioning sensor cable 210 can be achieved. Additionally, a receiving groove 113 is provided at the transition area between the long side surface 124 and the short side surface 125 of the fusion cage body 110 for receiving a clamping tool (not shown) of the fusion cage body 110 when implanting the interbody fusion device 100 or removing the fusion cage body 110. Certainly, the receiving groove 113 may also be provided at other positions on the side surface of the fusion cage body 110 as the position of the hole 112 changes. Preferably, the orientation of the receiving groove 113 and the orientation of the hole 112 are designed such that the orientation of the positioning sensor device 200 connected to the hole 112 is consistent with the orientation of the clamping tool clamped on the fusion cage body 110.

As shown in Fig. 2, at least one opening 114 is provided on a side surface of the fusion cage body 110 for positioning on a calibration device for calibrating the reference frame, particularly the interbody fusion device 100. The at least one opening 114 is connected to a second protrusion 303 on the calibration device, which will be further described below, in a form-fitting, plug-in, or clamping manner. If only one such opening 114 is provided on the side surface of the fusion cage body 110, and the cross section of the opening 114 is non-circular, preferably polygonal or elliptical, then the positioning of the reference frame, especially the interbody fusion cage of the present disclosure, by the calibration device for calibration position can be ensured with a minimum number of openings 114, i.e., one opening 114, on the side surface of the fusion cage body 110. This is because the non-circular, preferably polygonal or elliptical cross section of the opening 114 can restrict the rotation of the interbody fusion device 100 mounted thereon around the second protrusion 303 through form-fitting connection with the corresponding second protrusion 303 on the fusion cage body 110. The design of only one such shaped opening 114 is particularly advantageous when the fusion cage body 110 is small and the space or position for providing the opening 114 is very limited. If two or more openings 114 for positioning on the calibration device 300 for the reference frame are provided on the side surface of the fusion cage body 110, and the cross section of the openings 114 is annular or circular, the openings 114 that are easier to machine can be obtained, and thus the fusion cage body 110 that is easier to manufacture can be obtained.

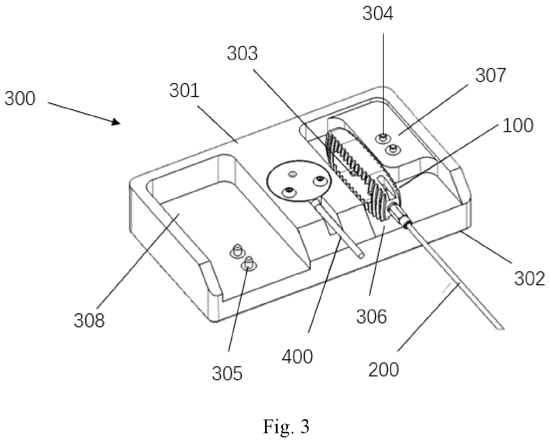

Fig. 3 is a perspective view of a calibration device 300 for the reference frame according to a non-limiting embodiment. The calibration device 300 according to the present disclosure may in principle be designed in any form as long as it can accommodate and position the reference frame and a calibration sensor device 400. As shown in the figure, the calibration device 300 may include a recess for accommodating the reference frame, a calibration sensor device 400 for calibration, and a positioning device for positioning the reference frame, where the reference frame, especially the interbody fusion cage, is mounted in the calibration device 300. For the vivid description of the method for calibrating a reference frame of the present disclosure, in the present disclosure, by way of example, the calibration device 300 is designed in the form of a substantially rectangular parallelepiped box having a top side and a bottom side. At least one recess with a quantity corresponding to that of the reference frames to be calibrated, for example three recesses with different sizes, is provided on the top side, where the rightmost in Fig. 3 shows a first recess 307, the middle shows a second recess 306 for accommodating the interbody fusion device 100 of the present disclosure, and the leftmost shows a third recess 308. The first recess 307 and the third recess 308 are respectively designed for accommodating reference frames with different sizes, styles or shapes from the interbody fusion device 100. The depths of the first recess 307, the second recess 306 and the third recess 308 are designed to correspond to the heights of the corresponding reference frames. Positioning devices for positioning the reference frame on the calibration device 300 are respectively provided in the first recess 307, the second recess 306 and the third recess 308, in particular at their bottoms. The positioning devices in the first, second and third recesses 304, 306, 308 may each be designed as at least one first protrusion 304, a second protrusion 303, and a third protrusion 305, respectively, where the shapes and sizes of these protrusions are designed to connect with openings in the side surfaces of the reference frames for fixation on the calibration device in a form-fitting manner. In particular, in the present embodiment, the shapes and sizes of the two second protrusions 303 are designed to be connected with the two openings 114 in the side surface of the fusion cage body 110 in a form-fitting manner, a plug-in manner or a clamping manner.

It is usually necessary to select an interbody fusion device 100 with a suitable size and shape according to the actual surgical conditions, which determines the size of the interbody fusion device 100 implanted into the human body, especially the space on the fusion cage body 110 for providing the openings 114 matched with the second protrusions 303, and the number of the second protrusions 303 can ultimately be determined according to the size of this space. If the space of the fusion cage body 110 for providing the openings 114 matched with the second protrusions 303 is limited, the positioning device may be designed as only one second protrusion 303, and the cross section of this second protrusion 303 is non-circular, preferably polygonal or elliptical, thereby ensuring the positioning of the reference frame, especially the interbody fusion device 100 of the present disclosure, by the calibration device 300 with a minimum number of second protrusions 303, i.e., one second protrusion 303. This is because the non-circular, preferably polygonal or elliptical cross section of the second protrusion 303 can restrict the rotation of the interbody fusion device 100 mounted thereon around the second protrusion 303 through form-fitting connection with the opening 114 in the fusion cage body 110. If the space of the fusion cage body 110 for providing the openings 114 matched with the second protrusions 303 is sufficiently large, the positioning device may be designed as two or more second protrusions 303, and the cross sections of these second protrusions 303 may be circular or annular. In the present embodiment, the second protrusions 303 may in particular be designed in the form of positioning pins, which makes the machining of the second protrusions 303 simpler.

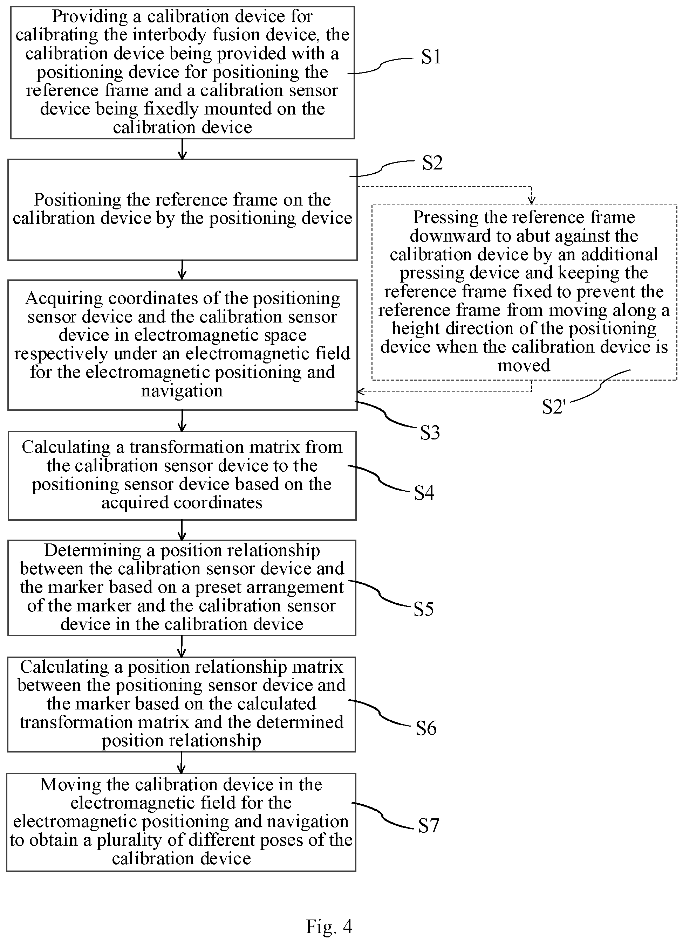

Fig. 4 shows a flow chart of steps of a method for calibrating the above-mentioned reference frame, especially the interbody fusion device 100, according to the present disclosure, where the dashed boxes represent optional steps and the solid boxes represent necessary steps of the method according to the present disclosure. The method for calibrating the reference frame according to the present disclosure includes the steps of:
S1: providing a calibration device 300 for the reference frame, the calibration device 300 being provided with a positioning device for positioning the reference frame, especially the interbody fusion device 100, the positioning device being designed as, for example, at least one second protrusion 303, and a calibration sensor device 400 being fixedly mounted on the calibration device 300, and a position relationship matrix between the positioning sensor device 200 and the marker 121 being determinable by the calibration device 300 to obtain the coordinates of the marker 121 in the electromagnetic space.
S2: positioning the reference frame, especially the interbody fusion device 100, on the calibration device 300 by the positioning device, where after positioning the reference frame, step S2' indicated by a dashed box in Fig. 4 may be additionally performed: pressing the reference frame, especially the interbody fusion device 100, downward to abut against the calibration device 300 by an additional pressing device and keeping the reference frame fixed to prevent the reference frame, especially the interbody fusion device 100, from moving along the height direction of the positioning device when the calibration device 300 is moved.
S3: acquiring the coordinates of the positioning sensor device 200 and the calibration sensor device 400 in the electromagnetic space respectively under an electromagnetic field for electromagnetic positioning and navigation.
S4: calculating a transformation matrix from the calibration sensor device 400 to the positioning sensor device 200 based on the acquired coordinates.
S5: determining a position relationship between the calibration sensor device 400 and the marker 121 based on a preset arrangement of the marker 121 and the calibration sensor device 400 in the calibration device 300.
S6: calculating a position relationship matrix between the positioning sensor device 200 and the marker 121 based on the calculated transformation matrix and the determined position relationship.

As a supplement, to reduce the error of the calculated position relationship matrix and thus further improve the electromagnetic positioning precision of the marker 121, after calculating a position relationship matrix between the positioning sensor device 200 and the marker 121, i.e., after completing step S6, step S7 represented by a dashed box in Fig. 4 may be additionally performed: moving the calibration device 300 in the electromagnetic field for electromagnetic positioning and navigation, where the movement may be translation or rotation, but according to the present disclosure, rotation is preferred, to obtain a plurality of different poses of the calibration device 300, where steps S3-S6 are repeated in each pose to acquire a plurality of position relationship matrices between the positioning sensor device 200 and the marker 121, and an average position relationship matrix is calculated from the plurality of position relationship matrices.

While examples of the present disclosure are provided in the foregoing description, modifications and variations may be made to these examples by those skilled in the art without departing from the scope and spirit of the present disclosure. For example, it should be understood that features of the embodiments herein may be applied to other embodiments described herein. Accordingly, the description is intended to be illustrative rather than restrictive. The present disclosure is defined by the appended claims, and all changes to the present disclosure that come within the meaning and range of equivalency of the claims are to be embraced within their scope.

## Claims

1. A reference frame for electromagnetic positioning and navigation of an orthopedic surgical robot, the reference frame being configured as an interbody fusion device, the reference frame comprising:
a fusion cage body, configured to be implanted between two vertebral bodies associated with a lesion, wherein the fusion cage body has a top surface, a bottom surface and a side surface, and an opening for filling bone graft material is provided in a middle area between the top surface and the bottom surface;
a positioning sensor device for the electromagnetic positioning and navigation of the orthopedic surgical robot, wherein the positioning sensor device is detachably connected to the interbody fusion device; and
a marker, configured to display a coordinate position in an image acquired by a medical imaging device, wherein the marker is fixedly arranged on the fusion cage body.

2. The reference frame according to claim 1, wherein the marker is designed as at least three tantalum markers, and the at least three tantalum markers are dispersedly arranged on the fusion cage body as a marking portion.

3. The reference frame according to claim 2, wherein the marker is designed as four tantalum markers.

4. The reference frame according to claim 2 or 3, wherein the top surface of the fusion cage body and the bottom surface of the fusion cage body are each at least partially provided with an anti-slip portion for increasing surface frictional resistance of contact between the two vertebral bodies and the fusion cage body.

5. The reference frame according to claim 4, wherein the anti-slip portion is designed as inverted teeth, which are uniformly distributed on the entire top surface and the entire bottom surface of the fusion cage body.

6. The reference frame according to claim 1 or 2, wherein a hole for detachably connecting the positioning sensor device is provided on the side surface of the fusion cage body, and the connecting is made in a friction fit manner.

7. The reference frame according to claim 1, wherein at least one opening for positioning on a calibration device for calibrating the reference frame is provided on the side surface of the fusion cage body, and the at least one opening is connected to a protrusion on the calibration device in a form-fitting manner, a plug-in manner or a clamping manner.

8. The reference frame according to claim 7, wherein only one opening is provided on the side surface of the fusion cage body, and a cross section of the only one opening is non-circular.

9. The reference frame according to claim 7, wherein two or more openings are provided on the side surface of the fusion cage body, and cross sections of the two or more openings are circular.

10. A method for calibrating the reference frame according to any one of claims 1 to 9, the method comprising steps:
S1: providing a calibration device for calibrating the interbody fusion device, the calibration device being provided with a positioning device for positioning the reference frame and a calibration sensor device being fixedly mounted on the calibration device;
S2: positioning the reference frame on the calibration device by the positioning device;
S3: acquiring coordinates of the positioning sensor device and the calibration sensor device in electromagnetic space respectively under an electromagnetic field for the electromagnetic positioning and navigation;
S4: calculating a transformation matrix from the calibration sensor device to the positioning sensor device based on the acquired coordinates;
S5: determining a position relationship between the calibration sensor device and the marker based on a preset arrangement of the marker and the calibration sensor device in the calibration device; and
S6: calculating a position relationship matrix between the positioning sensor device and the marker based on the calculated transformation matrix and the determined position relationship.

11. The method according to claim 10, wherein after completing step S6, step S7 is performed: moving the calibration device in the electromagnetic field for the electromagnetic positioning and navigation to obtain a plurality of different poses of the calibration device, wherein steps S3-S6 are repeated in each pose to acquire a plurality of position relationship matrices between the positioning sensor device and the marker, and an average position relationship matrix is calculated from the plurality of position relationship matrices.

12. The method according to claim 10 or 11, wherein the calibration device has a top side and a bottom side, at least one recess for accommodating at least one reference frame is provided on the top side of the calibration device, a depth of the least one recess is designed to correspond to a height of the reference frame, and the positioning device is provided in the at least one recess.

13. The method according to claim 12, wherein after step S2 and before step S3, step S2' is performed: pressing the reference frame downward to abut against the calibration device by an additional pressing device and keeping the reference frame fixed to prevent the reference frame from moving along a height direction of the positioning device when the calibration device is moved.

14. The method according to claim 10 or 11, wherein the positioning device is designed as at least one protrusion, a shape of the at least one protrusion and a size of the at least one protrusion are designed to be connected to at least one opening provided on the side surface of the fusion cage body in a form-fitting manner, a plug-in manner or a clamping manner.

15. The method according to claim 14, wherein the positioning device is designed as only one protrusion, and a cross section of the protrusion is non-circular.

16. The method according to claim 14, wherein the positioning device is designed as two or more protrusions, and cross sections of the two or more protrusions are circular or annular.
